# EUROPEAN PATENT APPLICATION

(11) **EP 3 093 015 A1**
(43) Date of publication of application: **16.11.2016**
(21) Application number: 16168701.7
(22) Date of filing: 09.05.2016
(51) Int. Cl.: A61K 31/352, A61K 36/062, A61K 36/185, A61P 25/28

(54) **COMPOSITION FOR ALLEVIATING AND PREVENTING ALZHEIMER'S DISEASE**

(30) Priority: 15.05.2015 TW 104115491 U
(71) Applicant: Sunway Biotech Co., Ltd., Taipei City 114, (TW)
(72) Inventor: Pan, Tzu-Ming, 114 Taipei City (TW); Lee, Chun-Lin, 114 Taipei City (TW); Lin, Pei-Ying, 114 Taipei City (TW); Hsu, Ya-Wen, 114 Taipei City (TW)
(74) Representative: Becker Kurig Straus

(57) **Abstract**

The present invention provides a composition for alleviating and preventing Alzheimer's disease. This composition comprises at least one yellow pigment extracted from a red mold product, and the said yellow pigment is Monascin or Ankaflavin. Moreover, after completing a variety of experiments, the composition has been proved possessing effects on alleviating and preventing AD by alleviating the symptoms of memory loss and learning disability resulted from the β-amyloid accumulated in brain of rats as well as reducing the inflammation and the oxidative stress caused by the β-amyloid in cerebral cortex and hippocampus tissue. Therefore, the experimental results have proved that this novel composition is indeed able to treat and prevent Alzheimer's disease.

## Description

### BACKGROUND OF THE INVENTION

### 1. Field of the Invention

The present invention relates to the technology field of medically-used composition, and more particularly to a composition for alleviating and preventing Alzheimer's disease.

### 2. Description of the Prior Art

Alzheimer's disease (AD), also called senile dementia disease, is a chronic neurodegenerative disease. The most common early symptom is difficulty in remembering recent events (short-term memory loss) when a person suffers from Alzheimer's disease. As the disease advances, Alzheimer's disease may show symptoms of problems with language, disorientation (including easily getting lost), mood swings, loss of motivation, not managing self care, and behavioural issues.

One of the hallmarks of Alzheimer's disease is the accumulation of amyloid plaques between nerve cells (neurons) in the brain. Amyloid is a general term for protein fragments, and Beta-amyloid is a protein fragment snipped from an amyloid precursor protein. In a healthy brain, these protein fragments are broken down and eliminated. In Alzheimer's disease, the fragments accumulate to form hard, insoluble plaques. Neurofibrillary tangles are insoluble twisted fibers found inside the brain's cells. These tangles consist primarily of a protein called Tau, which forms part of a structure called a microtubule. The microtubule helps transport nutrients and other important substances from one part of the nerve cell to another. In Alzheimer's disease, however, the Tau protein is abnormal and the microtubule structures collapse.

Among the numerous neurochemical abnormalities described for the brains of AD patients, the decrease in the activity of acetylcholine (AcCho)synthesizing enzyme, choline acetyltransferase (ChAT), is the most prominent and provides an excellent biochemical correlate of the severity of dementia in the disorder. These findings support the contention that cholinergic neurons are particularly vulnerable in AD brain and are consistent with the importance of AcCho in memory and learning processes.

FDA has approved 5 kinds of drugs for alleviating AD, wherein Acetylcholinesterase inhibitor is employed to reduce the rate at which acetylcholine (ACh) is broken down, thereby increasing the concentration of ACh in the brain and combating the loss of ACh caused by the death of cholinergic neurons. Moreover, Tacrine (Cognex®) and Donepezil (Aricept®) can also improve the memory loss of AD patient by increasing the content of Acetylcholine. However, because both Tacrine and Donepezil may cause side-effects such as headache and insomnia to AD patient, these two drugs cannot be the medication to cure AD.

Thus, because the commercial AD drugs may cause side-effects to AD patient, the inventor of the present application has made great efforts to make inventive research thereon and eventually provided a composition for alleviating and preventing Alzheimer's disease.

### SUMMARY OF THE INVENTION

The primary objective of the present invention is to provide a composition for alleviating and preventing Alzheimer's disease. This composition at least contains a yellow pigment extracted from a red mold product, and the said yellow pigment is Monascin or Ankaflavin. Moreover, after completing a variety of experiments, the composition has been proved possessing effects on alleviating and preventing AD by improving the symptoms of memory loss and learning disability resulted from the β-amyloid accumulated in brain of rats as well as reducing the inflammation and the oxidative stress caused by the β-amyloid in cerebral cortex and hippocampus tissue. Therefore, the experimental results have proved that this novel composition is indeed able to alleviate and prevent Alzheimer's disease.

In order to achieve the primary objective of the present invention, the inventor of the present invention provides a first embodiment of the composition for alleviating and preventing Alzheimer's disease, comprising at least one pure substance, and the pure substance is Monascin extracted from a red mold product; wherein a daily dosage of the composition for an adult user is above 18 mg.

According to the aforesaid first embodiment of the composition for alleviating and preventing Alzheimer's disease, wherein the composition can reduce the level of Malondialdehyde (MDA) in Cortex and Hippocampus by 1.5-2.9 times and 1.3-1.5 times, respectively.

According to the aforesaid first embodiment of the composition for alleviating and preventing Alzheimer's disease, wherein the composition can reduce the concentration of Reactive Oxygen Species (ROS) in Cortex and Hippocampus by 1.1-1.18 times and 1.20-1.61 times, respectively.

According to the aforesaid first embodiment of the composition for alleviating and preventing Alzheimer's disease, wherein the composition can reduce the activity of β-site APP-cleaving enzyme (BACE)in Cortex and Hippocampus by 1.15∼1.5 times and 1.44∼2.46 times, respectively.

According to the aforesaid first embodiment of the composition for alleviating and preventing Alzheimer's disease, wherein the composition can reduce the activity of apolipoprotein E (ApoE) in Cortex and Hippocampus by 1.22∼2.3 times and 1.71∼2.46 times, respectively.

According to the aforesaid first embodiment of the composition for alleviating and preventing Alzheimer's disease, wherein the composition can reduce the activity of Tau protein in Cortex and Hippocampus by 1.54∼2.35 times and 1.46∼2.2 times, respectively.

According to the aforesaid first embodiment of the composition for alleviating and preventing Alzheimer's disease, wherein the composition can reduce the accumulation of Beta-Amyloid 40 (Aβ40) in Cortex.

According to the aforesaid first embodiment of the composition for alleviating and preventing Alzheimer's disease, wherein the composition can reduce the accumulation of secreted amyloid precursor protein-α (sAPPα) in Cortex.

Moreover, for achieving the primary objective of the present invention, the inventor of the present invention provides a second embodiment of the composition for alleviating and preventing Alzheimer's disease, comprising at least one pure substance, and the pure substance is Ankaflavin extracted from a red mold product; wherein a daily dosage of the composition for an adult user is above 2.6 mg.

According to the aforesaid second embodiment of the composition for alleviating and preventing Alzheimer's disease, wherein the composition can reduce the level of Malondialdehyde (MDA) in Cortex and Hippocampus by 1.5∼2.9 times and 1.3∼1.5 times, respectively.

According to the aforesaid second embodiment of the composition for alleviating and preventing Alzheimer's disease, wherein the composition can reduce the concentration of Reactive Oxygen Species (ROS) in Cortex and Hippocampus by 1.1∼1.18 times and 1.20∼1.61 times, respectively.

According to the aforesaid second embodiment of the composition for alleviating and preventing Alzheimer's disease, wherein the composition can reduce the activity of β-site APP-cleaving enzyme (BACE) in Cortex and Hippocampus by 1.15∼1.5 times and 1.44∼2.46 times, respectively.

According to the aforesaid second embodiment of the composition for alleviating and preventing Alzheimer's disease, wherein the composition can reduce the activity of apolipoprotein E (ApoE) in Cortex and Hippocampus by 1.22∼2.3 times and 1.71∼2.46 times, respectively.

According to the aforesaid second embodiment of the composition for alleviating and preventing Alzheimer's disease, wherein the composition can reduce the activity of Tau protein in Cortex and Hippocampus by 1.54∼2.35 times and 1.46∼2.2 times, respectively.

According to the aforesaid second embodiment of the composition for alleviating and preventing Alzheimer's disease, wherein the composition can reduce the accumulation of Beta-Amyloid 40 (Aβ40) in Cortex.

According to the aforesaid second embodiment of the composition for alleviating and preventing Alzheimer's disease, wherein the composition can reduce the accumulation of secreted amyloid precursor protein-α (sAPPα) in Cortex.

### BRIEF DESCRIPTION OF THE DRAWINGS

The invention as well as a preferred mode of use and advantages thereof will be best understood by referring to the following detailed description of an illustrative embodiment in conjunction with the accompanying drawings, wherein:
FIG. 1 shows a chemical structure diagram of a yellow pigment extracted from a red mold product;
FIG. 2 shows a flow chart diagram including manufacturing processing steps for producing the red mold product;
FIG. 3 shows a flow chart diagram including the yellow pigment extracting steps;
FIG. 4 shows an experiment grouping table;
FIG. 5 shows a daily schedule diagram for animal experiment;
FIG. 6A shows a schematic framework view of a water maze used in a reference memory test;
FIG. 6B shows a four quadrants planning diagram of the water maze;
FIG. 7 shows a statistical bar plot of escape latency;
FIG. 8 shows swimming route records of test rats as working spatial navigation test;
FIG. 9 shows a statistical bar plot of time spent for searching target quadrant;
FIG. 10 shows a statistical bar plot of escape latency;
FIG. 11 shows a statistical bar plot of MDA level;
FIG. 12 shows a statistical bar plot of ROS concentration;
FIG. 13A shows a statistical bar plot of expression of BACE protein in Cortex;
FIG. 13B shows a statistical bar plot of expression of ApoE protein in Cortex;
FIG. 13C shows a statistical bar plot of expression of Tau protein in Cortex;
FIG. 14A shows a statistical bar plot of expression of BACE protein in Hippocampus;
FIG. 14B shows a statistical bar plot of expression of ApoE protein in Hippocampus;
FIG. 14C shows a statistical bar plot of expression of Tau protein in Hippocampus;
FIG. 15 shows several Aβ protein accumulation images of Cortex; and
FIG. 16 shows sAPPα accumulation images of Cortex.

### DETAILED DESCRIPTION OF THE PREFERRED EMBODIMENTS

To more clearly describe a composition for alleviating and preventing Alzheimer's disease according to the present invention, embodiments of the present invention will be described in detail with reference to the attached drawings hereinafter.

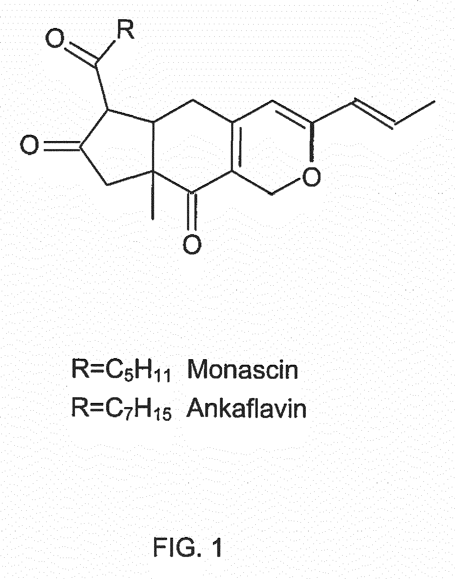

With reference to FIG. 1, there is shown a chemical structure diagram of a yellow pigment extracted from a red mold product. The present invention provides a composition for alleviating and preventing Alzheimer's disease. This composition is a yellow pigment shown in FIG. 1, and the said yellow pigment is Monascin or Ankaflavin extracted from a red mold product such as red mold rice (RMR) and red mold *Dioscorea* (RMD), wherein both the Monascin with a first daily dosage of above 18 mg and the Ankaflavin with a second daily dosage of above 2.6 mg possess effects on alleviating and preventing AD by alleviating the symptoms of memory loss and learning disability resulted from the β-amyloid accumulated in brain of rats as well as reducing the inflammation and the oxidative stress caused by the β-amyloid in cerebral cortex and hippocampus tissue.

Moreover, for an elderly people, to intake the Monascin by the first daily dosage or Ankaflavin by the second daily dosage is able to effectively control the accumulation of β-Amyloid and reduce the concentration of Reactive Oxygen Species (ROS) in Cortex and Hippocampus of brain, so as to achieve a preventive effect on from suffering Alzheimer's disease.

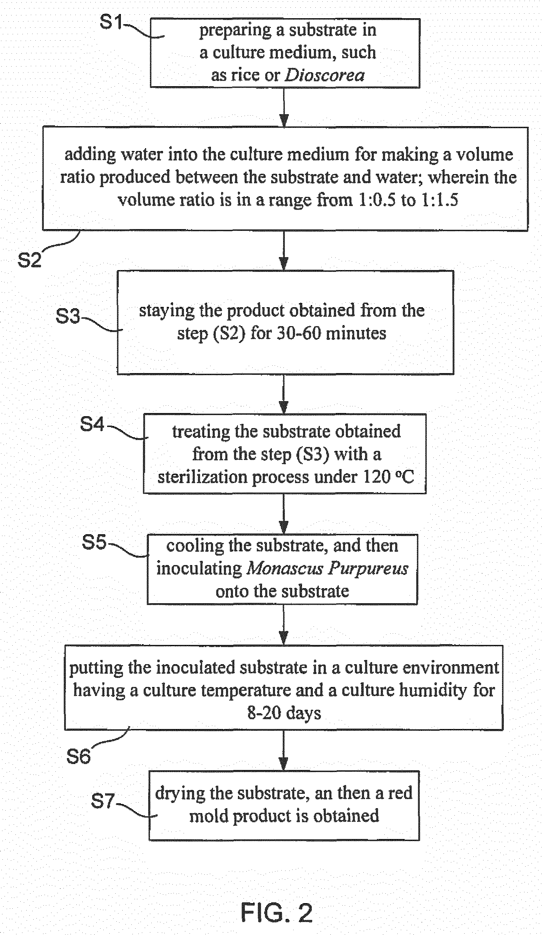

Please refer to FIG. 2, there is shown a flow chart diagram including manufacturing processing steps for producing the red mold product. As FIG. 2 shows, the aforesaid red mold product (RMR or RMD) is fabricated through following processing steps:
step (SI): preparing a substrate in a culture medium, such as rice or *Dioscorea*;
step(S2): adding water into the culture medium for making a volume ratio produced between the substrate and water; wherein the volume ratio is in a range from 1:0.5 to 1:1.5, and the best volume ratio is 1:0.75;
step(S3): staying the product obtained from the step (S2) for 30-60 minutes;
step(S4): alleviating the substrate obtained from the step (S3) with a sterilization process under 120 °C;
step(S5): cooling the substrate, and then inoculating *Monascus Purpureus* onto the substrate;
step(S6): putting the inoculated substrate in a culture environment having a culture temperature and a culture humidity for 8-20 days; wherein the culture temperature is in a range from 25 °C to 37 °C, and the culture humidity is in a range from 50% to 80%;
step (S7): drying the substrate, an then a red mold product is obtained.

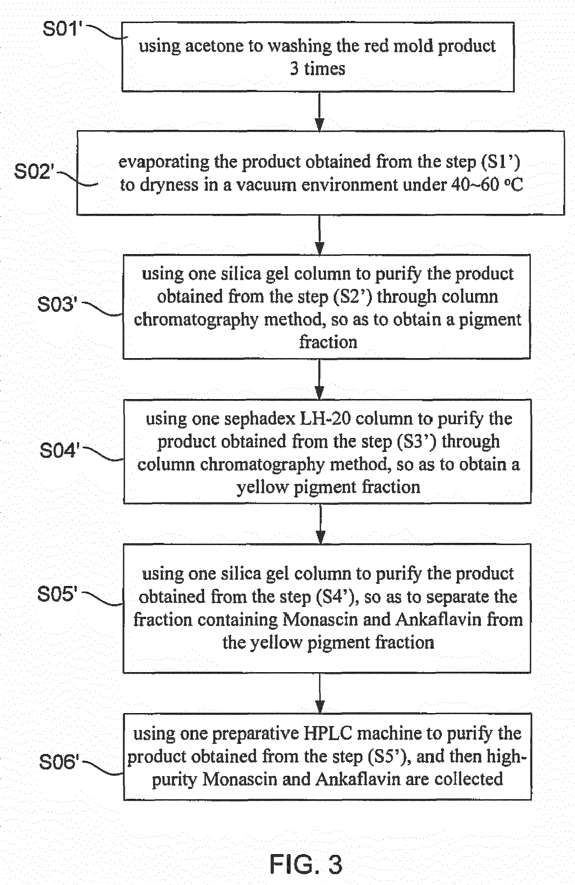

Please continuously refer to FIG. 3, where a flow chart diagram including a plurality of yellow pigment extracting steps is provided. As FIG. 3 shows, after obtaining the red mold product, it needs to extract Monascin and/or Ankaflavin from the red mold product through following extracting steps:
Step (S1'): using acetone to washing the red mold product 3 times;
step (S2'): evaporating the product obtained from the step (S1') to dryness in a vacuum environment under 40∼60 °C;
step (S3'): using one silica gel column to purify the product obtained from the step (S2') through column chromatography method, so as to obtain a pigment fraction;
step (S4'): using one sephadex LH-20 column to purify the product obtained from the step (S3') through column chromatography method, so as to obtain a yellow pigment fraction;
step (S5'): using one silica gel column to purify the product obtained from the step (S4'), so as to separate the fraction containing Monascin and Ankaflavin from the yellow pigment fraction;
step (S6'): using one preparative HPLC machine to purify the product obtained from the step (S5'), and then high-purity Monascin and Ankaflavin are collected.

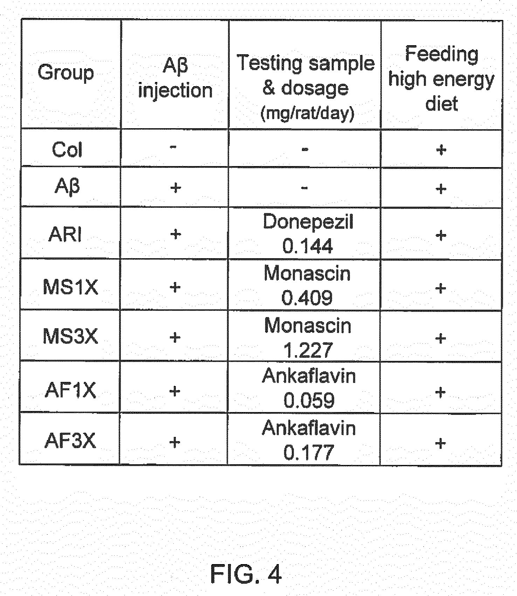

Therefore, above descriptions have completely introduce the fabricating way for this Alzheimer's disease alleviating and preventing composition. Next, a variety of experimental data will be presented in following paragraphs for proving the practicability of the Alzheimer's disease alleviating and preventing composition. Please refer to FIG. 4, which shoes an experiment grouping table. As the experiment grouping table presents, 8-week-old SD rats are divided into 7 experimental group, including:
(1) control (Col) group: consisting of normal SD rats, and the normal SD rats are fed with high energy diet;
(2)β-Amyloid (Aβ) group: consisting of several SD rats, wherein a β-Amyloid solution is injected into the SD rats' brain, and the SD rats are fed with high energy diet;
(3) Aricept (Ari) group: consisting of several SD rats, wherein a β-Amyloid solution is injected into the SD rats' brain, and the SD rats are fed with high energy diet and Donepezil of 0.144 mg;
(4) one-fold Monascin (MS 1X) group: consisting of several SD rats, wherein a β-Amyloid solution is injected into the SD rats' brain, and the SD rats are fed with high energy diet and Monascin of 1 fold dosage (0.409 mg);
(5) three-fold Monascin (MS3X) group: consisting of several SD rats, wherein a β-Amyloid solution is injected into the SD rats' brain, and the SD rats are fed with high energy diet and Monascin of 3 fold dosage (1.227 mg);
(6) one-fold Ankaflavin (AK1X) group: consisting of several SD rats, wherein a β-Amyloid solution is injected into the SD rats' brain, and the SD rats are fed with high energy diet and Ankaflavin of 1 fold dosage (0.059 mg);
(7) three-fold Ankaflavin (AK3X) group: consisting of several SD rats, wherein a β-Amyloid solution is injected into the SD rats' brain, and the SD rats are fed with high energy diet and Ankaflavin of 3 fold dosage (0.177 mg).

According to the body surface area (BSA) equation provided by Food and Drug Administration (FDA), the BSA of a standard man with the body height of 170 cm and the body weight of 65 kg can be calculated through the mathematical calculation formula of BSA (m²)=0.003207{H^{0.3}×W^{[0.0728S5(0.0188×LOG (w))}]}, wherein the BSA value obtained from aforesaid calculation formula for the standard man is 1.762 m². On the other hand, the BSA value for the SD rats can also be calculated through the mathematical calculation formula of BSA (m²) = (8.99W^{0.6899})/100, and the BSA value is 0.049 m². Moreover, because the animal experiments respectively use "18mg" and "2.6 mg" as the standard feeding dosage for Monacin and Ankaflavin, the "one-fold" dosage of Monacin and Ankaflavinin the experiment grouping table of FIG. 4 can be respectively calculated to 0.409 mgkg⁻¹day⁻¹and 0.059 mgkg⁻¹day⁻¹ according to BSA value of the SD rats. Herein, it needs to further explain that, Ari group is taken as a positive control group because Donepezil is a well-known drug for alleviating Alzheimer's disease. In the animal experiments, the lowest effective dosage (i.e., one-fold dosage) for Donepezil is defined to 0.144 mgkg⁻¹day⁻¹.

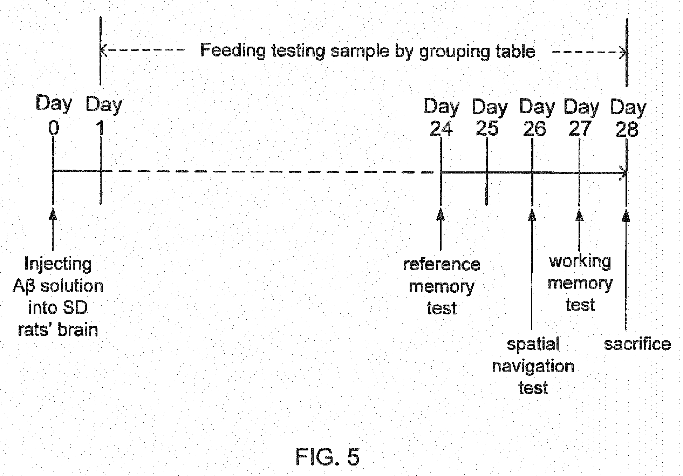

Please continuously refer to FIG. 5, where a daily schedule diagram for the animal experiments are provided. As FIG. 5 shows, the daily schedule for the animal experiments is designed and arranged as follows:
Day 0: injecting β-Amyloid 40 (Aβ 40) solution into SD rats' brain for making the SD rats become Aβ-induced AD (Alzheimer's disease) rats;
Day 1: starting to feed the rats in each of the experimental groups with corresponding test samples and high energy diets according to the experiment grouping table shown in FIG. 4;
Day 24: letting the rats in each of the experimental groups carry out reference memory test;
Day 26: letting the rats in each of the experimental groups carry out spatial probe test;
Day 27: letting the rats in each of the experimental groups carry out working memory test;
Day 28: sacrificing the rats in each of the experimental groups for taking their brains out, so as to analyze MDA level, ROS concentration, BACE activity, ApoE activity, P-Tau activity in Cortex and Hippocampus.

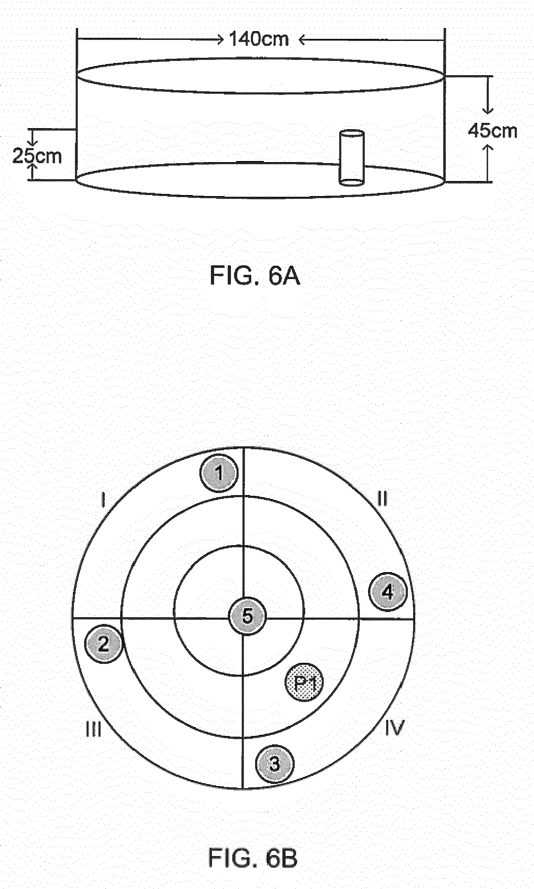

Please refer to FIG. 6A, which shows a schematic framework view of a water maze used in a reference memory test. The water maze 20 used for executing the reference memory test comprises: a circular tank with diameter of 140 cm and height of 45 cm; and a movable escape platform (or called rest platform) with diameter of 12 cm and height of 25 cm. Before starting the reference memory test, the circular tank needs to be filled with water to a specific height of 27 cm. Please simultaneously refer to FIG. 6B, which illustrates shows a four quadrants planning diagram of the water maze. As FIG. 6B shows, the circular tank is divided into four quadrants (I, II, III and IV). In addition, there have five starting positions set in the tank, and the location of the escape platform is marked as PI in the four quadrants planning diagram.

During the reference memory test, a camera is set at the ceiling above the center of the water tank for recording swimming routes of the rats. To carry out first-time reference memory test, the test rat is firstly put on the starting point 1 with the head outwards, and then letting the test rat take a break for 30 seconds if the rat find the escape platform in 90 seconds; eventually, the rat is put back into the feeding cage. Continuously, taking the test rat out of the feeding cage and putting the rat on next start point 2 for completing second-time reference memory test. Thereafter, repeating the same test steps to finish all reference memory tests by putting the test rat on others starting points (i.e., starting points 3 and 4).

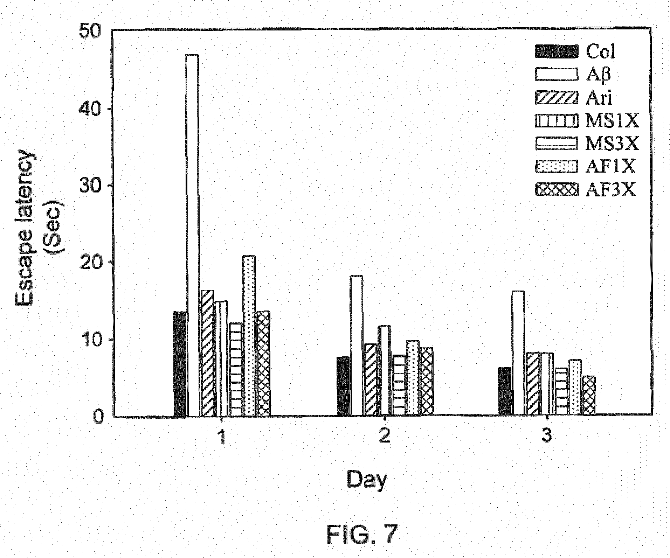

Please refer to FIG. 7, where a statistical bar plot of escape latency is shown. According to the experimental results shown in FIG. 7, it can find that, the rats of Col group have normal memory ability, and the rats of Aβ group must take a long time to find the rest platform. Moreover, comparing to the rats of Aβ group, the rats of MS1X, MS3X, AF1X, and AF3X groups spend less time to find the rest platform.

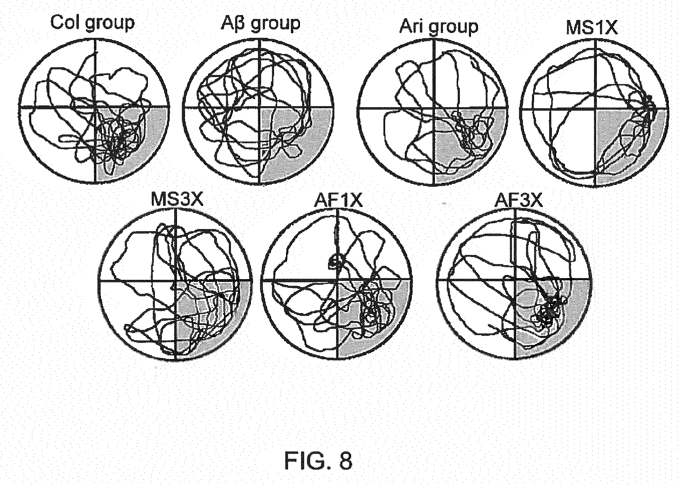

Next, spatial probe test is completed on Day 26. During the spatial probe test, the rest platform is removed out of the water tank. To carry out the spatial probe test, the test rat is put on the starting point 1 located in quadrant I; and then, letting the rat swim in the water tank for 90 seconds so as to record the rat's swimming route as well as the time for staying in quadrant IV. Please refer to FIG. 8, which provides swimming route records of the test rats as working the spatial navigation test. According to the swimming route records of FIG. 8, the swimming route of the Aβ-induced AD rats of the Aβ group shows the Aβ-induced AD rats does swim non-directionally and aimlessly during the spatial probe test. On the contrary, the rats of Col, Ari, MS1X, MS3X, AK1X, and AK3X groups directly swim into the quadrant IV (i.e., the target quadrant) in order to find the rest platform.

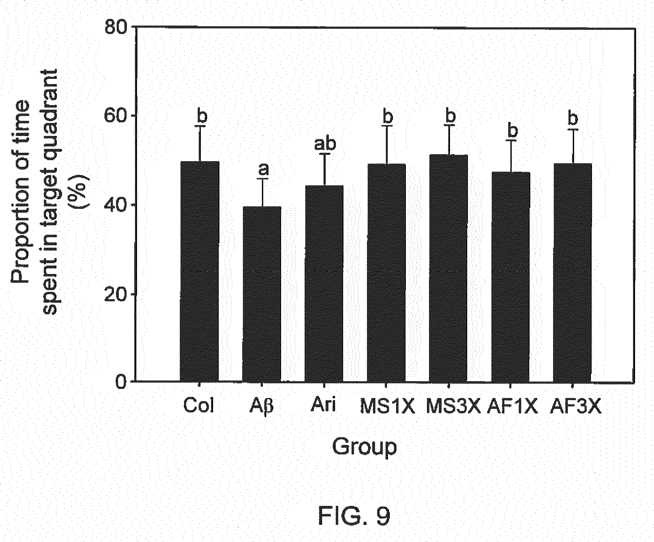

Reference memory test and spatial probe test are used to evaluate the memory and learning ability of the rats. Please refer to FIG. 9, there is shown a statistical bar plot of time spent for searching target quadrant. According to the statistical data provided by FIG. 9, it can find that, comparing to the rats of Col, Ari, MS1X, MS3X, AK1X, and AK3X groups, the Aβ-induced AD rats of the Aβ group spend more time to find the target quadrant (i.e., the quadrant IV). So that, the experimental data of FIG. 8 and FIG. 9 have proved that, both Monascin of 1-fold dosage and Ankaflavin of 1-fold dosage are able to alleviating the symptoms of memory loss and learning disability resulted from the β-amyloid accumulated in brain of rats.

Next, working memory test is completed on Day 27. During the working memory test, the rest platform is disposed in quadrant III. To carry out first-time working memory test, the test rat is firstly put on the starting point 1 with the head outwards, and then letting the test rat take a break for 15 seconds if the rat find the escape platform in 90 seconds; eventually, the rat is taken out of the water tank. Continuously, the rat is put on next start point 2 for completing second-time reference memory test. Thereafter, repeating the same test steps to finish all reference memory tests by putting the test rat on others starting points (i.e., starting points 3, 4, and 5).

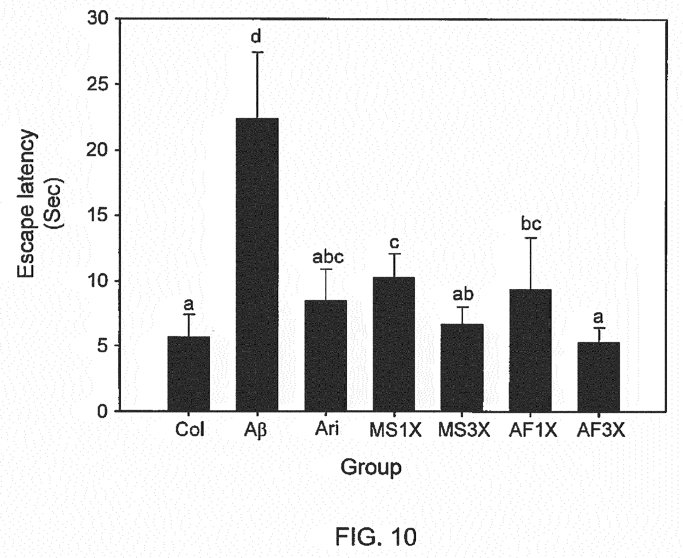

Please refer to FIG. 10, there is shown a statistical bar plot of escape latency. According to the statistical data provided by FIG. 10, it can find that, comparing to the rats of Col, Ari, MS1X, MS3X, AK1X, and AK3X groups, the Aβ-induced AD rats of the Aβ group spend more time to find the target quadrant (i.e., the quadrant IV). So that, the experimental data of FIG. 10 have proved that, both Monascin of 1-fold dosage and Ankaflavin of 1-fold dosage are able to alleviating the symptoms of memory loss resulted from the β-amyloid accumulated in brain of rats.

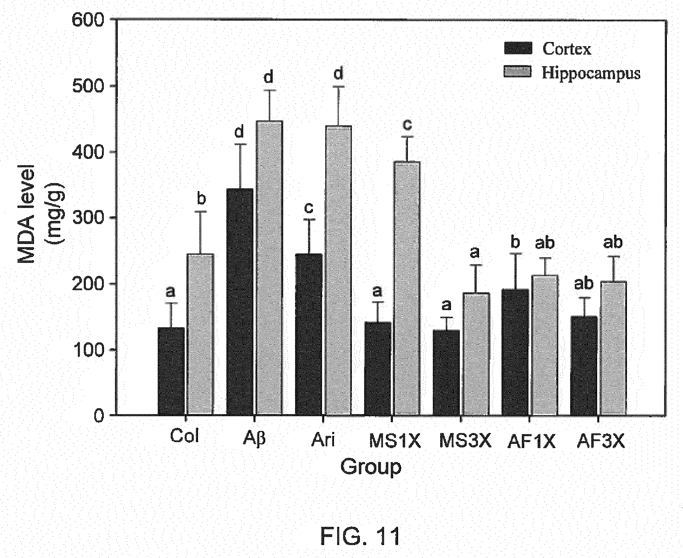

On DAY 28, the rats are eventually sacrificed in order to take out their brains for analyzing MDA level, ROS concentration, BACE activity, ApoE activity, P-Tau activity in Cortex and Hippocampus. Please refer to FIG. 11, where a statistical bar plot of MDA level is provided. As FIG. 11 shows, the Malondialdehyde (MDA) level in Cortex of the Aβ-induced AD rats of the Aβ group is higher than the rats' MDA level in Cortex of the Col group by 2.9 times. However, comparing to the Aβ-induced AD rats, the MDA level in Cortex of the rats of theMS1X, MS3X, AF1X, and AF3X groups are reduced by 1.5∼2.9 times. On the other hand, as FIG. 11 shows, the MDA level in Hippocampus of the Aβ-induced AD rats of the Aβ group is higher than the rats' MDA level in Hippocampus of the Col group by 1.8 times. However, comparing to the Aβ-induced AD rats, the MDA level in Hippocampus of the rats of theMSIX, MS3X, AF1X, and AF3X groups are reduced by 1.3∼1.5 times.

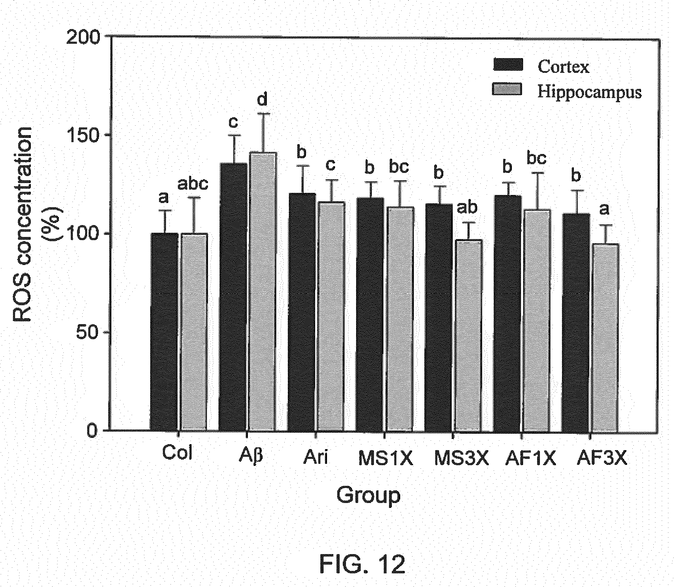

Continuously, please refer to FIG. 12, where a statistical bar plot of ROS concentration is provided. As FIG. 12 shows, the Reactive Oxygen Species (ROS) concentration in Cortex of the Aβ-induced AD rats of the Aβ group is higher than the rats' ROS concentration in Cortex of the Col group by 1.75 times. However, comparing to the Aβ-induced AD rats, the ROS concentration in Cortex of the rats of the MS1X, MS3X, AF1X, and AF3X groups are reduced by 1.1~1.18 times. On the other hand, as FIG. 12 shows, the ROS concentration in Hippocampus of the Aβ-induced AD rats of the Aβ group is higher than the rats' ROS concentration in Hippocampus of the Col group by 1.45 times. However, comparing to the Aβ-induced AD rats, the ROS concentration in Hippocampus of the rats of the MS1X, MS3X, AF1X, andAF3X groups are reduced by 1.20∼1.61 times.

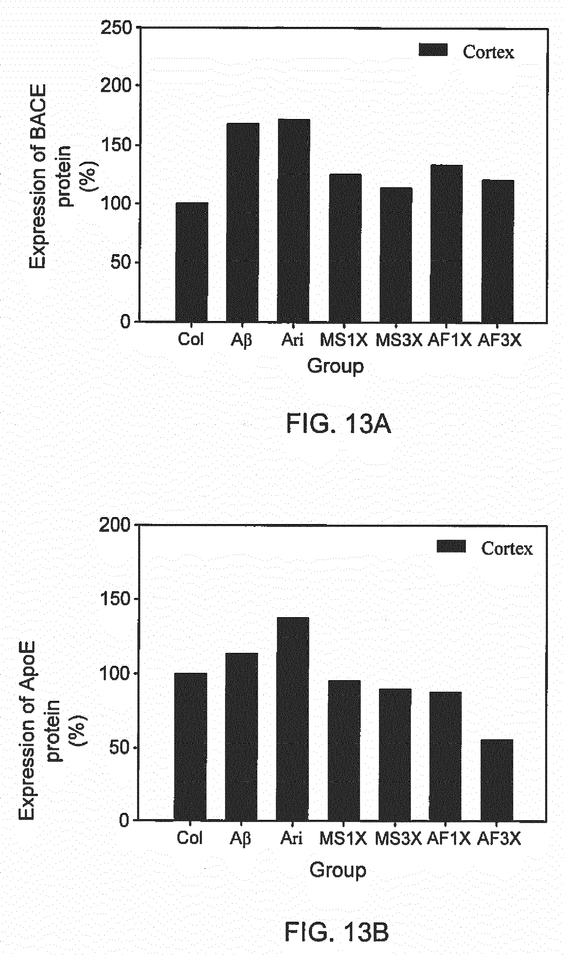

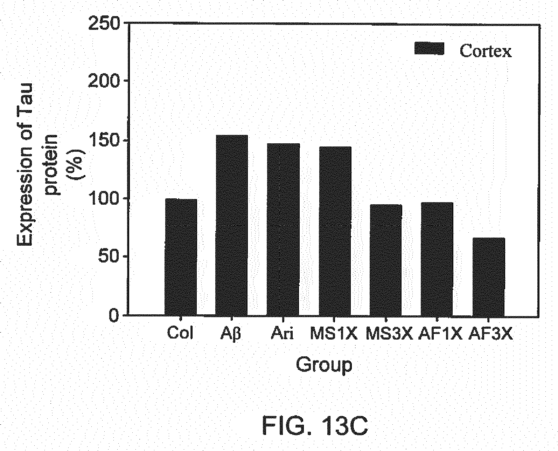

Please refer to FIG. 13A, where a statistical bar plot of expression of BACE protein in Cortex is provided. As FIG. 13A shows, the expression ofβ-site APP-cleaving enzyme (BACE) protein in Cortex of the Aβ-induced AD rats of the Aβ group is higher than the rats' BACE expression in Cortex of the Col group by 1.65 times. However, comparing to the Aβ-induced AD rats, the expression of BACE protein in Cortex of the rats of the MS1X, MS3X, AF1X, and AF3X groups are reduced by 1.15∼1.5 times. Please refer to FIG. 13B, which provides a statistical bar plot of expression of ApoE protein in Cortex. As FIG. 13B shows, the expression of Apolipoprotein E (ApoE) in Cortex of the Aβ-induced AD rats of the Aβ group is higher than the rats' ApoE expression in Cortex of the Col group by 1.2 times. However, comparing to the Aβ-induced AD rats, the expression of ApoE protein in Cortex of the rats of the MS1X, MS3X, AF1X, and AF3X groups are reduced by 1.22∼2.3 times. Moreover, please refer to FIG. 13C, which provides a statistical bar plot of expression of Tau protein (P-Tau) in Cortex. As FIG. 13C shows, the expression of Tau protein in Cortex of the Aβ-induced AD rats of the Aβ group is higher than the rats' P-Tau expression in Cortex of the Col group by 1.53 times. However, comparing to the Aβ-induced AD rats, the expression of Tau protein in Cortex of the rats of the MS1X, MS3X, AF1X, and AF3X groups are reduced by 1.54∼2.35 times.

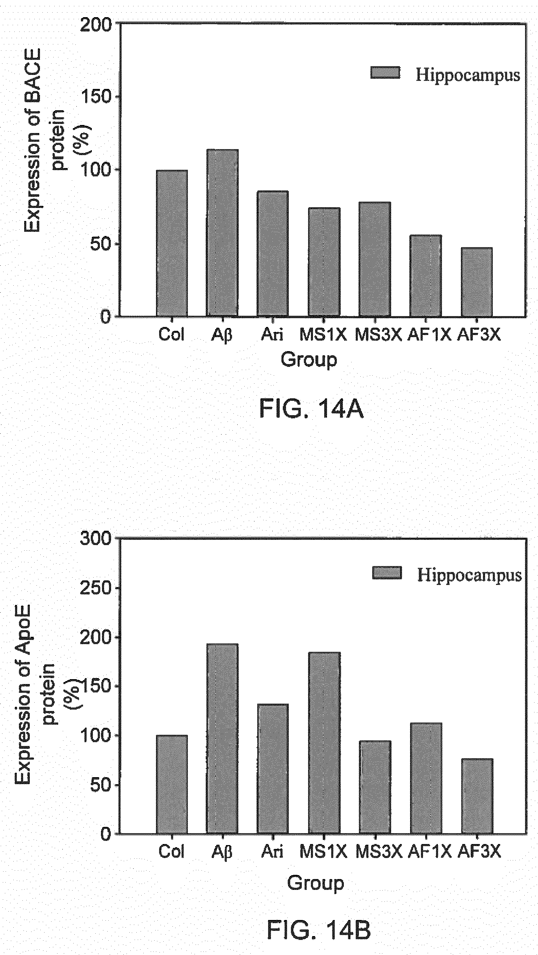

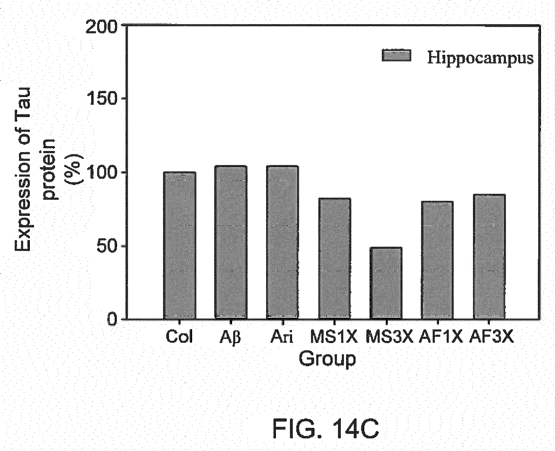

Please refer to FIG. 14A, where a statistical bar plot of expression of BACE protein in Hippocampus is provided. As FIG. 14A shows, the expression ofβ-site APP-cleaving enzyme (BACE) protein in Hippocampus of the Aβ-induced AD rats of the Aβ group is higher than the rats' BACE expression in Hippocampus of the Col group by 1.23 times. However, comparing to the Aβ-induced AD rats, the expression of BACE protein in Hippocampus of the rats of the MS1X, MS3X, AF1X, and AF3X groups are reduced by 1.44∼2.46 times. Please refer to FIG. 14B, which provides a statistical bar plot of expression of ApoE protein in Hippocampus. As FIG. 14B shows, the expression of Apolipoprotein E (ApoE) in Hippocampus of the Aβ-induced AD rats of the Aβ group is higher than the rats' ApoE expression in Hippocampus of the Col group by 1.97 times. However, comparing to the Aβ-induced AD rats, the expression of ApoE protein in Hippocampus of the rats of the MS1X, MS3X, AF1X, and AF3X groups are reduced by 1.71~2.46 times. Moreover, please refer to FIG. 13C, which provides a statistical bar plot of expression of Tau protein in Hippocampus. As FIG. 13C shows, the expression of Tau protein in Hippocampus of the Aβ-induced AD rats of the Aβ group is higher than the rats' P-Tau expression in Cortex of the Col group by 1.1 times. However, comparing to the Aβ-induced AD rats, the expression of Tau protein in Hippocampus of the rats of the MS1X, MS3X, AF1X, and AF3X groups are reduced by 1.46~2.2 times.

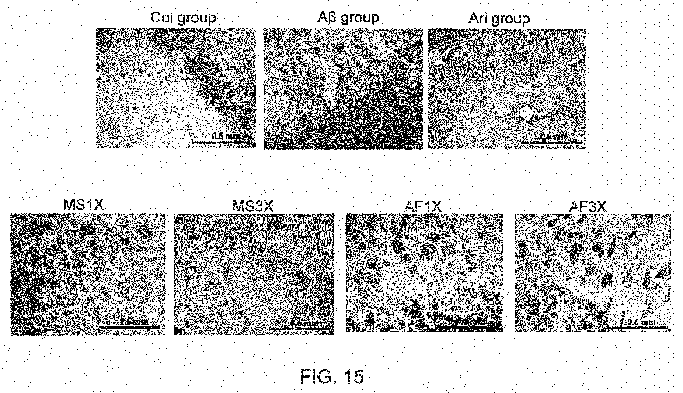

Continuously, please refer to FIG. 15, where several Aβ protein accumulation images of Cortex are shown. From FIG. 15, it can find that there has a large amount ofβ-Amyloid 40 (Aβ 40 protein) accumulating on Cortex of the AD rats of the Aβ group. On the contrary, comparing to the AD rats, the accumulation of Aβ 40 protein on Cortex of the rats of MS1X, MS3X, AF1X, and AF3X groups are obviously reduced.

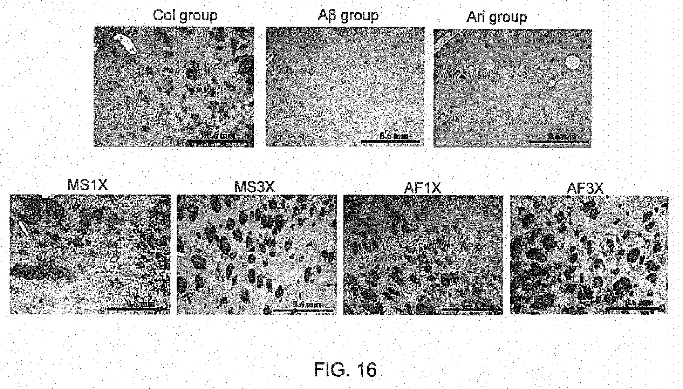

Moreover, please refer to FIG. 16, where several sAPPα accumulation images of Cortex are shown. From FIG. 16, it can find that there has a large amount of (ecreted amyloid precursor protein-α (sAPPα) accumulating on Cortex of the AD rats of the Aβ group. On the contrary, comparing to the AD rats, the accumulation of sAPPα on Cortex of the rats of MS1X, MS3X, AF1X, and AF3X groups are obviously reduced.

Therefore, through above descriptions, the composition for alleviating and preventing Alzheimer's disease provided by the present invention has been introduced completely and clearly; in summary, the present invention includes the advantages of:

(1) This Alzheimer's disease alleviating and preventing composition comprises at least one yellow pigment extracted from a red mold product, and the said yellow pigment is Monascin or Ankaflavin. Moreover, after completing a variety of experiments, the composition has been proved possessing effects on alleviating and preventing AD by alleviating the symptoms of memory loss and learning disability resulted from the A β 40 protein and sAPPα accumulated in brain of rats as well as reducing the inflammation and the oxidative stress caused by the accumulation of the two proteins in cerebral Cortex and Hippocampus tissue.

The above description is made on embodiments of the present invention. However, the embodiments are not intended to limit scope of the present invention, and all equivalent implementations or alterations within the spirit of the present invention still fall within the scope of the present invention.

## Claims

1. A composition for alleviating and preventing Alzheimer's disease, comprising at least one yellow pigment extracted from a red mold product; wherein the said yellow pigment is Monascin, and a daily dosage of the Monascin for an adult user to alleviate and prevent Alzheimer's disease is above 18 mg.

2. The composition for alleviating and preventing Alzheimer's disease of claim 1, wherein the composition can reduce the level of Malondialdehyde (MDA) in Cortex and Hippocampus by 1.5~2.9 times and 1.3∼1.5 times, respectively.

3. The composition for alleviating and preventing Alzheimer's disease of claim 1, wherein the composition can reduce the concentration of Reactive Oxygen Species (ROS) in Cortex and Hippocampus by 1.1~1.18 times and 1.20∼1.61 times, respectively.

4. The composition for alleviating and preventing Alzheimer's disease of claim 1, wherein the composition can reduce the activity of β-site APP-cleaving enzyme (BACE) in Cortex and Hippocampus by 1.15∼1.5 times and 1.44∼2.46 times, respectively.

5. The composition for alleviating and preventing Alzheimer's disease of claim 1, wherein the composition can reduce the activity of apolipoprotein E (ApoE) in Cortex and Hippocampus by 1.22∼2.3 times and 1.71~2.46 times, respectively.

6. The composition for alleviating and preventing Alzheimer's disease of claim 1, wherein the composition can reduce the activity of Tau protein in Cortex and Hippocampus by 1.54∼2.35 times and 1.46∼2.2 times, respectively.

7. The composition for alleviating and preventing Alzheimer's disease of claim 1, wherein the composition can reduce the accumulation of Beta-Amyloid 40 (Aβ40 protein) in Cortex.

8. The composition for alleviating and preventing Alzheimer's disease of claim 1, wherein the composition can reduce the accumulation of secreted amyloid precursor protein-α (sAPPα) in Cortex.

9. A composition for alleviating and preventing Alzheimer's disease, comprising at least one yellow pigment extracted from a red mold product; wherein the said yellow pigment is Ankaflavin, and a daily dosage of the Ankaflavin for an adult user to alleviate and prevent Alzheimer's disease is above 2.6 mg.

10. The composition for alleviating and preventing Alzheimer's disease of claim 9, wherein the composition can reduce the level of Malondialdehyde (MDA) in Cortex and Hippocampus by 1.5∼2.9 times and 1.3∼1.5 times, respectively.

11. The composition for alleviating and preventing Alzheimer's disease of claim 9, wherein the composition can reduce the concentration of Reactive Oxygen Species (ROS) in Cortex and Hippocampus by 1.1∼1.18 times and 1.20∼1.61 times, respectively.

12. The composition for alleviating and preventing Alzheimer's disease of claim 9, wherein the composition can reduce the activity of β-site APP-cleaving enzyme (BACE) in Cortex and Hippocampus by 1.15∼1.5 times and 1.44∼2.46 times, respectively.

13. The composition for alleviating and preventing Alzheimer's disease of claim 9, wherein the composition can reduce the activity of apolipoprotein E (ApoE) in Cortex and Hippocampus by 1.22∼2.3 times and 1.71∼2.46 times, respectively.

14. The composition for alleviating and preventing Alzheimer's disease of claim 9, wherein the composition can reduce the activity of Tau protein in Cortex and Hippocampus by 1.54∼2.35 times and 1.46∼2.2 times, respectively.

15. The composition for alleviating and preventing Alzheimer's disease of claim 9, wherein the composition can reduce the accumulation of Beta-Amyloid 40 (Aβ40 protein) in Cortex.

16. The composition for alleviating and preventing Alzheimer's disease of claim 9, wherein the composition can reduce the accumulation of secreted amyloid precursor protein-α (sAPPα) in Cortex.
